# EUROPEAN PATENT SPECIFICATION

(11) **EP 2 102 110 B1**
(45) Date of publication and mention of the grant of the patent: **28.11.2012**
(21) Application number: 07852160.6
(22) Date of filing: 28.02.2007
(51) Int. Cl.: C02F 1/26, A23L 1/30

(54) **OLIVE WASTE RECOVERY**
AUFBEREITUNG VON OLIVENABFALL
RÉCUPÉRATION DE DÉCHETS D'OLIVES

(30) Priority: 29.12.2006 SE 0602835
(43) Date of publication of application: 23.09.2009
(73) Proprietor: Phenoliv AB, 227 31 Lund (SE)
(72) Inventor: TORNBERG, Eva, S-227 31 lund (SE); GALANAKIS, Charis, 73100 Chania (GR)
(74) Representative: Allee, Harriet Eva Charlotta
(86) International application number: PCT/SE2007/001177
(87) International publication number: WO 2008/082343

(56) References cited:
- EP-A2- 1 310 175
- WO-A1-01/45514
- WO-A1-2005/123603
- ES-A1- 2 051 238
- FR-A1- 2 825 022
- GB-A- 2 415 136
- US-A1- 2003 031 740
- CARDOSO S.M. ET AL.: 'Calcium-mediated gelation of an olive pomace pectic extract' CARBOHYDRATE POLYMERS vol. 52, 2003, pages 125 - 133, XP004405540

## Description

### Technical field

The present invention relates to a method for isolating dietary fibres and valuable polyphenols from olive mill wastewater, as well as the use of said isolated products as additive in other food items and an anti-oxidant foodstuff, respectively.

### Background of the invention

There are three basic technologies used in olive oil extraction:
■ Pressing (traditional or classical system)
■ Centrifugal: three-phase (continuous system)
■ Centrifugal: two-phase (continuous system)

The manufacturing process of the centrifugal three-phase system usually yields: i) olive oil (20%), ii) solid waste (30%) and iii) aqueous liquor waste (50%). The solid waste (crude olive cake) is composed of a mixture of olive pulp and olive stones. The cakes are collected in central seed-oil extraction plants where the residual seed oil is extracted with hexane after being dried in rotary dryers using hot air of 60°C. Through this process there is an additional production of stones (exhausted olive cake), which are usually used as solid fuel [Niaounakis and Halvadakis, 2004].

In the pressing system and the centrifugal three-phase continuous system the waste, which will be the focus of this disclosure, is the 50 % liquid waste that is called olive mill wastewater (OMW). The quantity of OMW produced in the process ranges from 0.55 to 2 l/kg of olive, depending on the oil extraction process. Essentially, the OMW composition is water (80-83%), organic compounds, such as fibers, proteins, dyes and tannins (15-18%) and inorganic compounds (mainly potassium salts and phosphates) 2% although it varies broadly depending on many parameters such as olive variety, harvesting time, climate conditions, oil extraction process etc.

OMW is an acidic (usually pH 4.5 to 5) dark coloured liquid smelling strongly of oils. Unfortunately, it also contains phytotoxic and antibacterial substances like phenols, tannins and dyes, which prevent it from being disposed of. The presence of such substances causes OMW to be non-biodegradable and consequently unsuitable for further use as irrigation water [Niaounakis and Halvadakis, 2004]. Thereby, OMW is well known as a critical environmental problem in the Mediterranean area that accounts for ∼95% of the world olive oil production. Greece produces ∼1,500,000t of OMW annually, being the third olive oil producing country in the world after Spain and Italy. Although free disposal on the environment of the OMW is not permitted, it is estimated that most of the produced waste is discharged into rivers and land.

Efforts for OMW treatment have been developed over the last 50 years including recovery of valuable components, physical, thermal, physico-chemical and biological processes. None of the detoxification techniques on an individual basis permit the problem of disposal of OMW to be solved to a complete and exhaustive extent, effectively and in an ecologically satisfactory way. At the present state of the OMW treatment technology, industry has found no interest in supporting any traditional process on a wide scale. This is due to high investment and operational costs, the shortness of the production period (3-5 months) and the small size of the olive mills.

As discussed above, the OMW contains 15 to 18 % organic compounds. These organic compounds contain essentially fibres which come loose during the centrifugal three-phase continuous system. Most of the fibres are found in the cell wall material (CWM). The structure, type, size and shape of the cell walls differ and are closely linked to the function of the cell in a plant tissue. Generally, as a rule of thumb 30 % of the cell wall is considered to be cellulose, 30% pectin and 30% hemicellulose. Together with the remaining 10% (the non-polysaccharide polymer lignin and some proteins) they are involved in the creation of the rather intricate network building the cell wall.

Pectin is a natural polysaccharide that forms thick colloidal solutions in water. It can be added to processed foods to create texture. Medicinally, the compound has been widely used in antidiarrheal products, serving as a stool-forming agent. It also appears to lower blood lipoprotein levels. Citrus pectin (CLMP) is representative of typical commercial low-methoxyl pectin, used as a food additive. CLMP has a high galacturonic acid content and low levels of acetylation and neutral sugars. Although lemon and orange rinds are among the richest sources of the compound, containing up to 30% of this polysaccharide, pectin is also found in the roots and fruits of most plants, such as olives, which are known to contain about one third of arabinose-rich pectic polysaccharide. An olive pectin (OLMP) was extracted from olive pomace (waste produced by two-phase separation technology) and was characterized by a lower galacturonic acid content, higher acetylation degree, lower availability of free carboxylic groups due to the higher degree of methyl-esterification, lower MW and a higher content in neutral sugars, mainly arabinose (see Table 1).

**Table 1: Physico-chemical characteristics of the pectin samples [Cardoso et al., 2003].**

| | CLMP | OLMP |
|---|---|---|
| Degree of methyl-esterification | 34.8 % | 43.2% |
| Degree of acetylation | <1 % | 11 % |
| Galacturonic acid content | 88.7 % | 44.1% |
| Total neutral sugars | 4.8 % | 38.8 % |
| Ash | 1.7 % | 1.1 % |
| Calcium | 2.5 mg/g | 0.33 mg/g |
| Intrinsic viscosity (pH 7, 0.1 mol/l NaCl) | 2.62 dl/g | 1.04 dl/g |
| Average (viscometric) molecular weight (Mᵥ) | 5.13x10⁴ | 1.45x10⁴ |

| | | |
|---|---|---|
| Dry weight basis (when applied) | | |

Another valuable compound present in olives are the polyphenols. Polyphenols are a group of chemical substances found in plants, characterized by the presence of more than one phenol group per molecule. The polyphenols are responsible for the coloring of some plants-for example the color of leaves in the autumn. Research indicates that a class of polyphenols has antioxidant characteristics with potential health benefits. These polyphenol antioxidants may reduce the risk of cardiovascular disease and cancer, and it has been proposed that these substances reduce the onset of Alzheimer's. GB 241536 describes a method for extracting polyphenols from OMW using enzymes or ethanol during heating. It comprises filtration and concentration of the extract, and optionally a defatting step. There is no disclosure of recovering dietary fibres, and the extraction step uses only ethanol. Cardoso et al, 2003, discloses the pectin extraction from fresh pomace.

WO 01/45574 describes a method for preparing an antioxidant composition from OMW. However, there is no mention of the recovery of fibers.

The purpose of the technique presented in this patent is to present a relatively simple and low cost procedure to extract soluble dietary fibres and valuable polyphenols from the OMW that can be used as additives in other food items.

### Summary of the present invention

The object of the present invention is to isolate antioxidatively acting polyphenols as well as dietary fibres, such as pectins from olive waste material.

In particular the present invention relates to a process for isolating soluble dietary fibers and valuable polyphenols from olive mill wastewater, wherein the olive mill wastewater is defatted by removing the fat by centrifugation and concentrated by removing the water content. The remaining mixture is extracted using ethanol with an organic acid and the dietary fibers are separated from the polyphenols by precipitation of the dietary fibers in ethanol. The soluble dietary fibers are further separated from the insoluble dietary fibers by redissolving the precipitated dietary fibers into water whereby the insoluble dietary fibers precipitate leaving the soluble dietary fibers in solution.

In a preferred embodiment of the invention the defatted OMW is concentrated between 55 to 65 °C.

In a preferred embodiment of the invention the ethanol concentration of the extraction is up to 7 % (v/v).

In a further preferred embodiment of the invention the concentration of the organic acid used is up to 3 % by weight of the extraction solution.

In another preferred embodiment of the invention the organic acid used is present in an amount of at least 0.5 % by weight of the extraction solution.

In a further preferred embodiment of the invention the organic acid used is selected from the group consisting of citric acid, tartaric acid, malonic acid, maleic acid, malic acid, oxalic acid, adipic acid, and fumaric acid.

In a preferred embodiment of the invention the ethanol concentration used for the extraction of polyphenols is at least 85 % (v/v).

In a preferred embodiment of the invention the extracted polyphenols are separated from the dietary fibers by filtration.

In a preferred embodiment of the invention the extracted ethanol-rich liquid containing the polyphenols is diluted to a liquid containing 15-40 % ethanol.

In a preferred embodiment of the invention the diluted ethanol-rich liquid containing the polyphenols is clarified by filtration.

In a preferred embodiment of the invention the precipitated dietary fibers are redissolved into water at 1-4 % w/v.

In another preferred embodiment of the invention the soluble dietary fibers are separated from the insoluble dietary fibers by centrifugation.

In a preferred embodiment of the invention soluble fibers are concentrated by evaporation.

In a preferred embodiment of the invention the isolated polyphenol-rich liquid is used to produce a once-a-day administrable dose of antioxidatively acting polyphenols.

In a preferred embodiment of the invention the evaporated soluble fiber solution is used as an additive and /or a fat replacer in other food items.

### Brief description of the drawings

Figure 1 shows a schematic view of the isolation process for polyphenols and soluble dietary fibres from olive mill wastewater (OMW).
Figure 2 shows the evolution of methanol versus time at different heating temperatures of OMW

### Detailed description of the present invention

Procedure of extracting soluble dietary fibers and valuable polyphenols from olive mill waste (OMW).

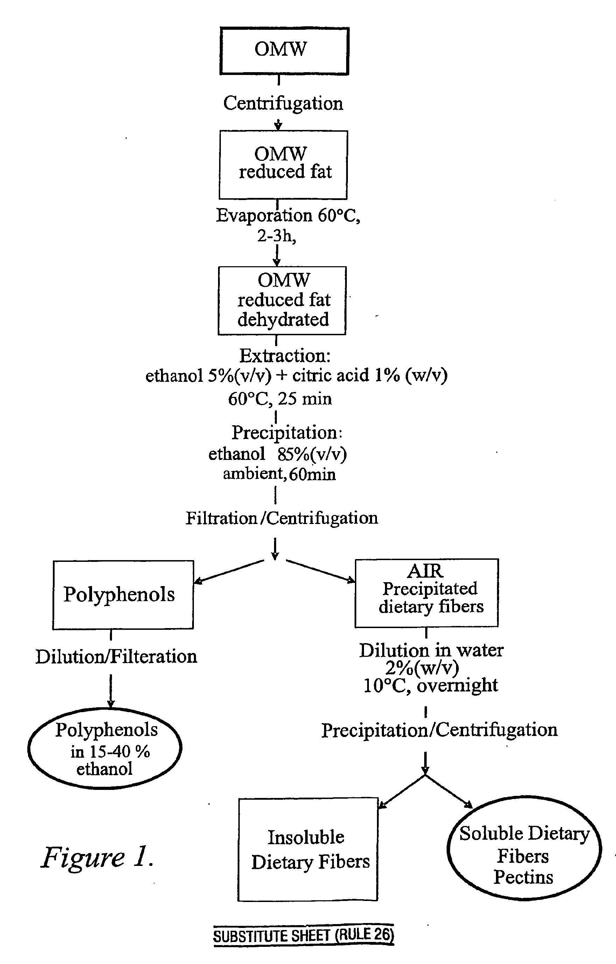

The following, including Figure 1 outlines a method for isolating both the soluble dietary fibers and the valuable polyphenols from olive mill wastewater (OMW) in the same process. OMW is collected from an olive mill industry and a typical content of OMW is given in Table 2a below.

**Table 2a. A typical content of OMW (Niaounakis M. & Halvadakis C. P. (2004))**

| Parameter | Unit | Value |
|---|---|---|
| Total Phenols | mg/L | 3 000-24 000 |
| Organic matter | g/L | 40-165 |
| Minerals | g/L | 5-14 |
| Solids | %w/w | 5.5-17.6 |
| pH | | 4.5-6 |

As can be seen from Table 2a the content varies broadly depending on parameters as discussed above. Thus, the content of the specific sample used to describe the invention herein is given in Table 2b below:

**Table 2b. OMW sample characterization**

| Parameter | Unit | Value |
|---|---|---|
| Total Phenols | mg/L | 6012±769 |
| COD | mg/L | 139063±21302 |
| Fats | % w/w | 0.89±0.06 |
| Fats | % dmb | 8.42±0.56 |
| Total Solids without fats | % w/w | 8.77±0.10 |
| Water content | % w/w | 90.35±0.04 |

The first step is to reduce the fat content of the OMW sample by 44 % from 8.42 % down to 4.7 % DS (Dry Substance) by a centrifugation step at 3000 g for 30 min.

Secondly, the water content of the OMW has to be reduced. This can preferably be conducted under air supply by a rotary evaporator at between 50 to 80°C. preferably between 55 to 65 °C and most preferably at 60°C for 30 to 180 min, more preferably between 60 min to 180 min and most preferably at about 120 min. As discussed above, the olive pectin when compared to commercial citrus pectin, is characterized by a lower galacturonic acid content, higher acetylation degree, and lower availability of free carboxylic groups due to the higher degree of methyl-esterification (Table 1). The purpose of concentrating at the elevated temperature of 60°C for about 2 hours is to activate pectin methyl esterase (PME) of the waste and partially hydrolyse the high-methoxy and high-acetyl pectin of olive wastewater. A comprehensive study of the optimal deactivation temperature is presented below. The concentrated sample (3.6 fold concentration) is cooled to room temperature and weighed.

The extraction of the fibres from the partly defatted and concentrated slurry of OMW is thereafter performed by adding ethanol to an ultimate concentration of no more than 7 % (v/v), preferably about 5 % (v/v) and between 0.5 - 3 % (w/v), preferably 1 % (w/v) of citric acid in the slurry. The extraction is performed by continuously stirring with the alcohol and the citric acid at between 60 to 80°C, preferably at 60 °C, for 25 min by heating the extraction bottle in a water bath.

In order to separate the dietary fibres from small mono- and disaccharides and polyphenols the extraction mixture is dispersed in ethanol at a final concentration of 85% (v/v) and maintained at ambient temperature for 1 hour. Precipitation of the dietary fibres (water soluble and insoluble) occurs and the mixture is cooled down to room temperature, and subsequently filtered through a G3 glass filter isolating an ethanol- rich residual liquid and the precipitate, named Alcohol Insoluble Residue (AIR). The filtered liquid had a nice yellowish colour and an attractive fruity smell of olives, which was evident when diluted to a liquid of 15 to 40 % ethanol.

The amount of polyphenols (expressed as Total Phenols) was measured at three different alcohol concentration, viz. 85 % (v/v), 40 % (v/v), and 25 % (v/v), respectively, after appropriate dilution with water. The results obtained are shown in the table 3 below. These ethanolic extracts are as rich in polyphenols as the extra virgil olive oils, which are known to contain up to 1000 ppm total phenols (Montedoro et al., 1992). Polyphenols are known as good anti-oxidants and therefore can be sold as a healthy and tasty additive to drinks. In one embodiment of the invention the polyphenol rich extract can be concentrated by membrane technology or rotary evaporator. In one embodiment of the invention the diluted polyphenol rich extract can be clarified by filtration.

**Table 3**

| Ethanol conc. after dilution % | Amount of extracted Total Phenols ppm |
|---|---|
| 85 | 1254 |
| 40 | 750 |
| 25 | 501 |

The amount of fibre expressed as AIR, the Alcohol Insoluble Residue, that is obtained after removing the mono-, disaccharides and polyphenols from the extract obtained in this way was 6.3 % based on the initial OMW sample, which is about 72 % of the available solids not including fat (see table 2b). The content of Total Phenols of the AIR can be determined, and was in this example 300 mg per 100 g of AIR (3000 ppm). The content of Total Fibers was calculated as the sum of nonstarch polysaccharides and ash-free acid insoluble residue (klason lignin) and accounted for 37.6 % (w/v) of the AIR. The nonstarch polysaccharide residues were determined to be 5.1 %, while the "Klason lignin" fraction including native lignin, tannins and Maillard reaction products (Theander et al., 1995) was accounted for 32.5 %.

In order to isolate the soluble fibres (pectins), the Alcohol Insoluble Residue (AIR) obtained in step 2 above was dissolved (1-4 % w/v) in water and stirred, using magnetic stirring of the solution overnight at 10°C. In alternate embodiment of the invention the precipitated dietary fibers are redissolved into a weak base solution (0.1 to 0.5 N sodium hydroxide or calcium dihydroxide solution) up to a pH value of 6, wherein the base de-esterifies the pectin and increase the pH value to optimize the pectin for use in meat products. In order to separate the soluble from the insoluble fibres, the extraction mixture was centrifuged for 20 min with 3000 g at 20°C. Alternatively the solution can be filtered.

This separation provided a supernatant taken as soluble pectin fibres (63.8 % (w/w) of the extract) and the precipitate as insoluble fibre (36.2 % (w/w)), respectively. In this example, the pH value was monitored to be 5.30 at this point. The degree of methyl-esterification (DM) of the soluble pectin material was calculated to be 59 %.

A highly concentrated olive pectin solution is isolated from the supernatant obtained during the centrifugation, alternatively from the filtrate in the filtration step above. The supernatant containing the soluble fibres of AIR is separated from the pellet and concentrated ∼2-3 times by using a rotary evaporator (60°C) and compressed air. The concentrated solution can be concentrated further by heating at 80°C for at least 1 h. The resultant solution of soluble fibre from OMW had a final pectin concentration of 7.2 % (w/w) and was allowed to stand for 48 h at 5°C to form a gel.

The ash content of these soluble fibres of the AIR was relatively high and equals to 44 % on a dry matter basis. Therefore the concentration of pectin in the solution is about 4 %. One way to remove the ash from the solution of soluble fibres and to further concentrate the sample is to use membrane filtration or any other suitable filtration and isolation technique.

The elastic modulus of the gels obtained was in the region of 30 Pa, which was measured using a constant stress rheometer and Oscillation Stress Sweep Tests (shear stress from 0 to 100 Pa and 1 Hz frequency). Cardoso et al., 2003 have measured the elastic modulus of Calcium-mediated gelation of an olive pomace pectic extract and they found values in the same range, i.e. from 10 to 100 Pa.

This gelling ability of soluble fibres from OMW can be used as an additive and /or a fat replacer in other food items.

The optimal temperature for the activation of pectin methyl esterase (PME) of the waste to partially hydrolyse the high-methoxy and high-acetyl pectin Pectin gels produced from high-methoxy pectin require acid conditions (near pH 3) and a high soluble solids content (65%) in order to produce satisfactory jelly. On the other hand, pectin gels produced from low-DM pectin are produced over a wide range of pH with or without addition of sugar in the presence of small portions of calcium (EI-Nawawi and Heikal, 1995). The degree of methyl-esterification can be controlled by the activity of PME (Anthon and Barrett, 2006). It is known that preheating vegetables to above 50 °C, in a so-called low-temperature blanch, activates PME (Van Buren et al., 1960).

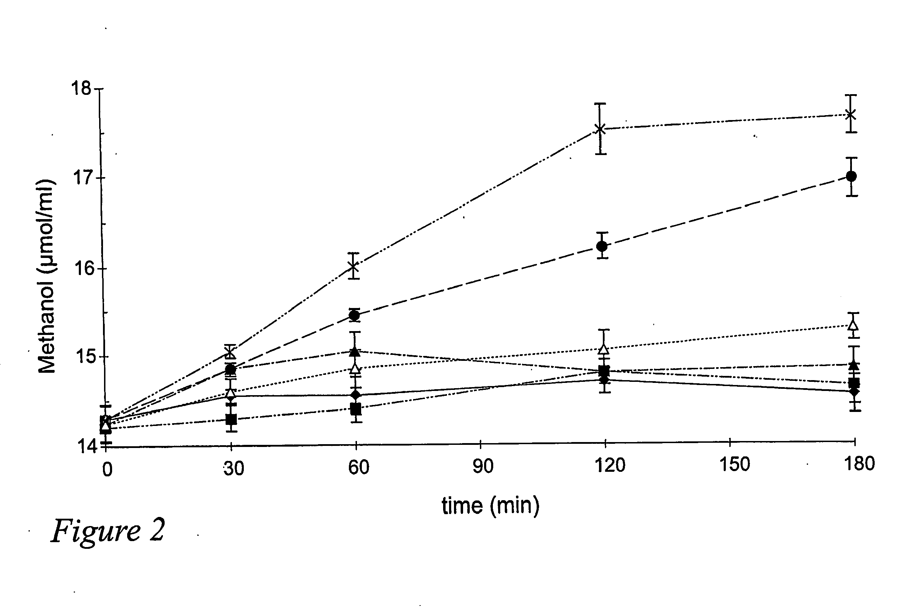

Figure 2 shows the evolution of methanol versus time at different heating temperatures of OMW: 50 (Δ), 55(●), 60(x), 65(▲), 70(■) and 80(◆) °C. The evolution of methanol gives an indication of possible pectin de-esterification and is measured as follows: Briefly, 100 µL of sample (diluted 1:25 in distilled water) are placed in 2 ml screw-cap plastic tubes and 180 µL of citrate buffer (100mM, pH = 6.5) was added. Subsequently, 20 µL of diluted alcohol oxidase (0.01 unit/µL in citrate buffer) and 100 µL of water was added in order to give a final volume of 400 µL. The tubes were gently mixed and incubated at 30 °C for 10 min. Subsequently, 400 µL of Purpald solution (5 mg/mL in 0.5 N NaOH, prepared within one hour) were added to each tube and the samples were capped and vigorously vortexed to ensure oxygenation. The tubes were placed in a water bath at 30 °C for 30 min. Samples are then removed from the water bath and 1.2 ml of distilled water was added for a final volume of 2.0 ml. Absorbance at 550 nm is then determined against a methanol standard curve (between 0 and 200 µmol/100ml). A blank sample was prepared with distilled water, citrate buffer and diluted alcohol oxidase.

Heating of OMW from 50 to 60 °C led to an accumulation of methanol, indicating possible pectin de-esterification. At this temperature range, the rate of de-estirification increases substantially with increasing temperature. Moreover, 2 h thermal treatment at 60 °C is adequate for endogenous PME activation in OMW samples. A determination of the methylation degree of the OMW pectin could establish that methanol release is originated from pectin de-esterification. Methanol accumulation was rather low and not significant for heating above 65 °C. A possible deactivation of PME occurs after treatment for 1 h and 30 min at 65-70 and 80 °C, respectively (Figure 2). Therefore, a possible heat treatment at 60 °C for 3 h could progress both deesterification of pectin and pectin solubization.

The thermal treated OMW mixtures were further processed for the recovery of the alcohol insoluble residue as described below. An aliquot of OMW sample heat treated (as above) was dispersed in ethanol up to a final concentration of 85% (v/v). Each mixture was stirred at 4 °C for 1 h, filtered and then extracted twice with chloroform/methanol (2:1, v/v, 3-fold initial fresh weight) for 30 min under continuous stirring.

Table 4 shows the quantity of the recovered AIR, the galacturonic acid content of AIR, the galacturonic acid content of water soluble AIR fraction as well as the corresponding degree of methylation of water soluble pectin.

**Table 4. Characteristics of recovered AIR from OMW treated samples for 3 h at different temperatures.**

| OMW Sample | AIR (w/w) | GalA** (w/w) | Water soluble GalA*** (w/w) | DM of water soluble pectin (%) |
|---|---|---|---|---|
| Raw | 2.7±0.1 | 4.9±0.2 | 6.2±0.2 | 78±2 |
| 50 °C | 2.8±0.1 | 5.0±0.1 | 7.6±0.2 | 73±1 |
| 55 °C | 2.7±0.1 | 4.9±0.1 | 8.2±0.1 | 62±2 |
| 60 °C | 2.7±0.1 | 4.8±0.1 | 8.7±0.2 | 59±2 |
| 65 °C | 2.8±0.1 | 5.0±0.1 | 7.8±0.2 | 64±1 |
| 70 °C | 2.8±0.1 | 4.8±0.2 | 7.6±0.1 | 65±1 |
| 80 °C | 2.7±0.1 | 4.8±0.1 | 7.3±0.1 | 71±1 |

| | | | | |
|---|---|---|---|---|
| * AIR is expressed as a percentage initial OMW sample. ** GALA is expressed as a percentage of AIR. *** GalA is expressed as a percentage of water soluble AIR. | | | | |

The recovered AIR quantity as well as the galacturonic acid content was not affected by the different applied thermal treatments. The degree of methylation of water soluble pectin decreased significantly from 78 to 59%, for 50 to 60 °C. The GalA of water soluble pectin was increased from 6.2 to 8.7%, for 50 to 60 °C. The results indicate the activation of PME at 60 °C, with a subsequent increase in solubilization of pectin material as well as a decrease in DM of the recovered pectin material.

### REFERENCES

Anthon G.E. and Barrett D.M. (2006). "Characterization of the temperature activation of pectin methylesterase in green beans and tomatoes". Journal of Agricultural and Food Chemistry, 54: 204-211.
Cardoso S.M., M.A. Coimbra and Lopez da Silva J.A. (2003). "Temperature dependence of the formation and melting of pectin-Ca2+ networks: a rheological study". Food Hydrocolloids, 17: 801-807.
Cardoso S.M., M.A. Coimbra and Lopez da Silva J.A. (2003). "Calcium-mediated gelation of an olive pomace pectic polysaccharide arabinan side chains". Carbohydrate Polymers, 52: 125-133.
EI-Nawawi S.A. and Heikalb Y.A. ( 1995). "Factors affecting the production of low-ester pectin gels". Carbohydrate Polymers, 26: 189-I 93.
Montedoro, G., Servili, M., Baldioli, M. and Miniati, E. (1992). "Simple and hydrolysable phenolic compounds in virgin olive oil. 1. Their extraction, separation, and quantitative and semiquantitative evaluation by HPLC". Journal of Agricultural and Food Chemistry, 40: 1571-1576.
Niaounakis M. and C.P. Halvadakis (2004). Olive-Mill waste management. Literature Review and Patent Survey, Typothito - George Dardanos.
Theander, O.P., Aman, E., Westerlund, R., and Petterson, D. (1995). "Total Dietary Fiber determined as neutral sugar residues, uronic acid residues and Klason lignin (The Uppsala Method): Collaborative Study". Journal of AOAC International, 78: 1030-1044.
Van Buren, J.P., J.C. Moyer, D. Wilson, W.B. Robinson and Hand D.B. (1960). "Influence of blanching conditions on sloughing, splitting, and firmness of canned snap beans". Food Technology, 14: 233-236.

## Claims

1. Process for isolating soluble dietary fibers and valuable polyphenols from olive mill wastewater, **characterized by** the following steps:
the olive mill wastewater is
(a) defatted by removing the fat by centrifugation and;
(b) concentrated by removing the water content and;
(c) extracted using ethanol and an organic acid and;
(d) the dietary fibers are separated from the polyphenols by precipitation of the dietary fibers in ethanol and;
(e) the soluble dietary fibers are separated from the insoluble dietary fibers by redissolving the precipitated dietary fibers into water whereby the insoluble dietary fibers precipitate leaving the soluble dietary fibers in solution.

2. Process according to claim 1, wherein the concentration of the wastewater in step (b) is occurred at 50 to 60°C, for 2 to 3 h.

3. Process according to claim 1, wherein the ethanol concentration of the extraction in step (c) is up to 7% (v/v).

4. Process according to claim 1, wherein the concentration of the organic acid used in step (c) is up to 3% by weight of the extraction solution.

5. Process according to claim 1, wherein the organic acid used in step (c) is present in an amount of at least 0.5% by weight of the extraction solution.

6. Process according to claim 1, wherein the organic acid used in step (c) is selected from the group consisting of citric acid, tartaric acid, malonic acid, maleic acid, malic acid, oxalic acid, adipic acid, and fumaric acid.

7. Process according to claim 1, wherein the ethanol concentration used in step (d) for the extraction of polyphenols is at least 85% (v/v).

8. Process according to claim 1, wherein the extracted polyphenols in step (d) are separated from the dietary fibers by filtration.

9. Process according to claim 8, wherein the extracted ethanol-rich liquid containing the polyphenols is diluted to a liquid containing 15-40% ethanol.

10. Process according to claim 9, wherein the diluted ethanol-rich liquid containing the polyphenols is clarified by filtration.

11. Process according to claim 1, wherein the precipitated dietary fibers in step (e) are redissolved into water at 1-4% w/v.

12. Process according to claim 1, wherein the soluble dietary fibers in step (e) are separated from the insoluble dietary fibers by centrifugation.

13. Process according to claim 12, wherein soluble fibers are concentrated by evaporation.

## Patentansprüche

1. Verfahren zum Isolieren von löslichen Ballaststoffen und wertvollen Polyphenolen aus Olivenölmühlen-Abwasser, **gekennzeichnet durch** die nachstehenden Schritte:
das Olivenölmühlen-Abwasser wird
(a) entfettet, indem das Fett **durch** Zentrifugieren entfernt wird; und
(b) **durch** Entfernen des Wassergehalts konzentriert; und
(c) unter Verwendung von Ethanol und einer organischen Säure extrahiert; und
(d) die Ballaststoffe werden von den Polyphenolen durch Ausfällung der Ballaststoffe in Ethanol getrennt; und
(e) die löslichen Ballaststoffe werden von den unlöslichen Ballaststoffen **durch** Wiederauflösen der ausgefällten Ballaststoffe in Wasser getrennt, wodurch die unlöslichen Ballaststoffe ausfallen und die löslichen Ballaststoffe in Lösung verbleiben.

2. Verfahren gemäss Anspruch 1, wobei das Konzentrieren des Abwassers in Schritt (b) bei 50 bis 60°C über 2 bis 3 Stunden durchgeführt wird.

3. Verfahren gemäss Anspruch 1, wobei die Ethanolkonzentration im Extraktionsschritt (c) bis zu 7 % (V/V) beträgt.

4. Verfahren gemäss Anspruch 1, wobei die Konzentration der in Schritt (c) verwendeten organischen Säure bis zu 3 Gew.% der Extraktionslösung beträgt.

5. Verfahren gemäss Anspruch 1, wobei die in Schritt (c) verwendete organische Säure in einer Menge von mindestens 0,5 Gew.% der Extraktionslösung vorliegt.

6. Verfahren gemäss Anspruch 1, wobei die in Schritt (c) verwendete organische Säure aus der Gruppe bestehend aus Zitronensäure, Weinsäure, Malonsäure, Maleinsäure, Äpfelsäure, Oxalsäure, Adipinsäure und Fumarsäure ausgewählt ist.

7. Verfahren gemäss Anspruch 1, wobei die in Schritt (d) verwendete Ethanolkonzentration zur Extraktion des Polyphenols mindestens 85 % (V/V) beträgt.

8. Verfahren gemäss Anspruch 1, wobei in Schritt (d) die extrahierten Polyphenole durch Filtration von den Ballaststoffen abgetrennt werden.

9. Verfahren gemäss Anspruch 8, wobei die extrahierte ethanolreiche Flüssigkeit, die die Polyphenole enthält, zu einer Lösung, enthaltend 15 bis 40 % Ethanol, verdünnt wird.

10. Verfahren gemäss Anspruch 9, wobei die verdünnte ethanolreiche Flüssigkeit, die die Polyphenole enthält, durch Filtration geklärt wird.

11. Verfahren gemäss Anspruch 1, wobei in Schritt (e) die ausgefällten Ballaststoffe in 1 bis 4 % (G/V) Wasser wieder aufgelöst werden.

12. Verfahren gemäss Anspruch 1, wobei in Schritt (e) die löslichen Ballaststoffe durch Zentrifugieren von den unlöslichen Ballaststoffen getrennt werden.

13. Verfahren gemäss Anspruch 12, wobei die löslichen Ballaststoffe durch Verdampfen konzentriert werden.

## Revendications

1. Procédé d'isolement de fibres alimentaires solubles et de polyphénols de valeur issus d'eaux usées d'un moulin à olives, **caractérisé par** les étapes suivantes :
les eaux usées du moulin à olives sont
(a) déshuilées en éliminant l'huile par centrifugation ; et
(b) concentrées en éliminant le contenu en eau ; et
(c) extraites en utilisant l'éthanol et un acide organique ; et
(d) les fibres alimentaires sont séparées des polyphénols par précipitation des fibres alimentaires dans l'éthanol ; et
(e) les fibres alimentaires solubles sont séparées de fibres alimentaires insolubles en redissolvant les fibres alimentaires précipitées dans de l'eau, moyennant quoi les fibres alimentaires insolubles précipitent, laissant les fibres alimentaires solubles en solution.

2. Procédé selon la revendication 1, dans lequel la concentration des eaux usées à l'étape (b) est effectuée à 50 à 60 °C, pendant 2 à 3 h.

3. Procédé selon la revendication 1, dans lequel la concentration en éthanol de l'extraction à l'étape (c) va jusqu' à 7 % (v/v).

4. Procédé selon la revendication 1, dans lequel la concentration en acide organique utilisée à l'étape (c) va jusqu'à 3 % en poids de la solution d'extraction.

5. Procédé selon la revendication 1, dans lequel l'acide organique utilisé à l'étape (c) est présent en une quantité d'au moins 0,5 % en poids de la solution d'extraction.

6. Procédé selon la revendication 1, dans lequel l'acide organique utilisé à l'étape (c) est choisi dans le groupe consistant en l'acide citrique, 1"acide tartrique, l'acide malonique, l'acide maléique, l'acide malique, l'acide oxalique, l'acide adipique et l'acide fumarique.

7. Procédé selon la revendication 1, dans lequel la concentration en éthanol utilisée à l'étape (d) pour l'extraction des polyphénols est d'au moins 85 % (v/v).

8. Procédé selon la revendication 1, dans lequel les polyphénols extraits à l'étape (d) sont séparés des fibres alimentaires par filtration.

9. Procédé selon la revendication 8, dans lequel le liquide riche en éthanol extrait contenant les polyphénols est dilué en un liquide contenant de 15 à 40 % d'éthanol.

10. Procédé selon la revendication 9, dans lequel le liquide riche en éthanol dilué contenant les polyphénols est clarifié par filtration.

11. Procédé selon la revendication 1, dans lequel les fibres alimentaires précipitées à l'étape (e) sont redissoutes dans de l'eau à hauteur de 1 à 4 % p/v.

12. Procédé selon la revendication 1, dans lequel les fibres alimentaires solubles à l'étape (e) sont séparées des fibres alimentaires insolubles par centrifugation.

13. Procédé selon la revendication 12, dans lequel les fibres solubles sont concentrées par évaporation.
